# EUROPEAN PATENT SPECIFICATION

(11) **EP 1 545 534 B1**
(45) Date of publication and mention of the grant of the patent: **18.04.2007**
(21) Application number: 03798175.0
(22) Date of filing: 23.09.2003
(51) Int. Cl.: A61K 31/502, A61P 7/00, A61P 7/06

(54) **USE OF 4-PYRIDYLMETHYL-PHTHALAZINE DERIVATIVES FOR THE MANUFACTURE OF A MEDICAMENT FOR THE TREATMENT OF MYELODYSPLASTIC SYNDROMES**
VERWENDUNG VON 4-PYRIDYLMETHYL-PHTHALAZIN-DERIVATEN ZUR HERSTELLUNG EINES MEDIKAMENTS ZUR BEHANDLUNG VON MYELODYSPLASTISCHEN SYNDROMEN
UTILISATION DE DERIVES DE 4-PYRIDYLMETHYL-PHTHALAZINE DANS LA PRODUCTION D'UN MEDICAMENT PERMETTANT DE TRAITER LES SYNDROMES MYELODYSPLASIQUES

(30) Priority: 24.09.2002 US 413176 P
(43) Date of publication of application: 29.06.2005
(73) Proprietor: Novartis AG, 4056 Basel (CH); Novartis Pharma GmbH, 1230 Wien (AT); The Arizona Board of Regents on behalf of the University of Arizona, Tucson, AZ 85724 (US)
(72) Inventor: DUGAN, Margaret, Han, Woodside, NY 11377 (US); LIST, Alan, Tucson, AZ 85718 (US)
(74) Representative: Grubb, Philip William
(86) International application number: PCT/EP2003/010578
(87) International publication number: WO 2004/028542

(56) References cited:
- WO-A-00/59509
- WO-A-01/10859
- WO-A-98/35958

## Description

The present invention relates to the use of a 4-pyridylmethyl-phthalazine derivative alone or in combination with further therapeutic agents, for example, those defined herein, for the preparation of a medicament for the treatment of myelodysplastic syndromes; especially myelodysplastic syndromes which are resistant to conventional chemotherapy, to a combination comprising a 4-pyridylmethyl-phthalazine derivative, a further therapeutic agent as defined herein and optionally at least one pharmaceutically acceptable carrier, for simultaneous, separate or sequential use; and to a pharmaceutical composition and a commercial package comprising said combination.

Many patients in the early stages of MDS experience no symptoms at all. A routine blood test will reveal a reduced red cell count (anemia) sometimes along with reduced white cell (neutropenia) and/or platelet counts (thrombocytopenia). On occasion the white cell and platelet counts maybe low while the hematocrit remains normal. The counts are not low enough to produce symptoms. Other patients experience definite symptoms. These symptoms depend on which blood cell type is involved, as well as the level to which the cell count drops.

For the diagnosis of MDS initially a complete blood cell count is done. If it is confirmed that a patient has a low hematocrit, possibly along with a low white cell and/or platelet count, a bone marrow examination is performed. The bone marrow is examined to define the percentage of blasts and abnormally mature cells (dysplastic cells). A sample is stained to determine iron content. A chromosomal analysis is also performed to discover any abnormalities (such as a missing or extra chromosome). Periodic bone marrow exams help determine whether MDS has transformed to AML.

For patients having a high risk of progression to AML intravenous chemotherapy is sometimes applied. Relatively strong doses of chemotherapy are given to "induce" control of the disease. Unfortunately, the chance of controlling MDS with induction chemotherapy with established chempotherapeutic agents is only about 30%. Even in successful cases, the disease often returns within twelve months. Furthermore, clinical heterogeneity and inadequate understanding of the disease pathobiology have limited progress in the development of therapeutics for the treatment of MDS. Therefore, there is a strong need for further chemotherapeutic agents for the treatment of MDS, especially chemotherapeutic agents having a higher rate of controlling MDS and/or prolonging the period until the disease returns.

Surprisingly, it was found that 4-pyridylmethyl-phthalazine derivatives are useful for the treatment of MDS.

4-Pyridylmethyl-phthalazine derivatives which a suitable for the present invention, their preparation and pharmaceutical formulations containing the same are described in WO00/59509, EP02/04892, WO01/10859 and, especially, in U.S. Patent No. 6,258,812.

4-Pyridylmethyl-phthalazine derivatives and, in particular 4-pyridylmethyl-phthalazine derivatives of formula I, 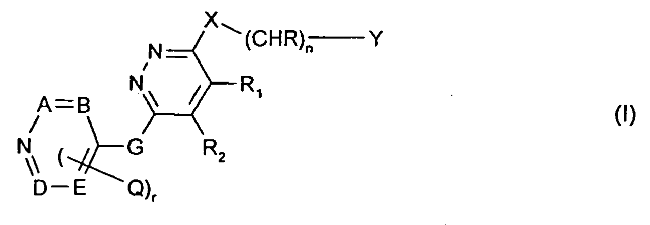 wherein the radicals and symbols have the meanings as defined below, the N-oxides of these 4-pyridylmethyl-phthalazine derivatives, as well as the salts thereof, are tyrosine kinase inhibitors, which were designed to inhibit the vascular endothelial growth factor (VEGF) signal transduction by binding directly to the ATP-binding sites of VEGF receptors. Such 4-pyridylmethyl-phthalazine derivatives reduce the microvasculature and inhibit growth of primary tumors and metastases in animal models and are useful for treating diseases associated with deregulated angiogenesis, especially neoplastic diseases (solid tumors), such as breast cancer, cancer of the colon, lung cancer, especially small cell lung cancer, and cancer of the prostate.

For example, PTK787 (1-(4-chloroanilino)-4-(4pyridylmethyl)phthalazine also known as ZK222584), a compound of formula I, wherein r, n and m are each 0, R₁ and R₂ together form a bridge of subformula I* (see below), A, B, D and E are each CH, G is methylene, X is imino, Y is 4-chlorophenyl, and the bonds characterized by a wavy line are double bonds, is most specific for KDR, but can also inhibit Flt-1 and Flt-4 and has activity against other tyrosine kinase receptors, including c-Kit.

Hence, the invention relates to the use of a 4-pyridylmethyl-phthalazine derivative for the preparation of a medicament for the treatment of MDS, especially MDS which is resistant to conventional chemotherapy, preferably the use of a 4-pyridylmethyl-phthalazine derivative of formula I, wherein
r is 0 to 2,
n is 0 to 2,
m is 0 to 4,
R₁ and R₂ (i)
together form a bridge in subformula I* 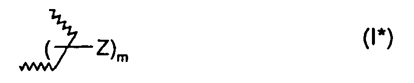
the binding being achieved via the two terminal carbon atoms, or
   (ii) together form a bridge in subformula I** 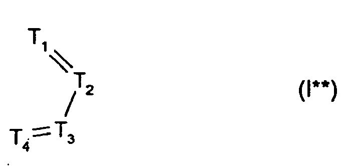
wherein one or two of the ring members T₁, T₂, T₃ and T₄ are nitrogen, and the others are in each case CH, and the binding is achieved via T₁ and T₄;
A, B, D, and E are, independently of one another, N or CH, with the stipulation that not more than 2 of these radicals are N;
G is lower alkylene, lower alkylene substituted by acyloxy or hydroxy, -CH₂-O-, -CH₂-S-, -CH₂NH-, oxa (-O-), thia (-S-), or imino (-NH-);
Q is lower alkyl;
R is H or lower alkyl;
X is imino, oxa, or thia;
Y is unsubstituted or substituted aryl, pyridyl, or unsubstituted or substituted cycloalkyl; and
Z is amino, mono- or disubstituted amino, halogen, alkyl, substituted alkyl, hydroxy, etherified or esterified hydroxy, nitro, cyano, carboxy, esterified carboxy, alkanoyl, carbamoyl, N-mono- or N,N-disubstituted carbamoyl, amidino, guanidino, mercapto, sulfo, phenylthio, phenyl-lower alkylthio, alkylphenylthio, phenylsulfonyl, phenyl-lower alkylsulfinyl or alkylphenylsulfinyl, substituents Z being the same or different from one another if more than 1 radical Z is present;
and wherein the bonds characterized, if present, by a wavy line are either single or double bonds;
or an N-oxide of the defined compound,
or the salt of such compound having at least one salt-forming group.

The radicals and symbols as used in the definition of a compound of formula I have the meanings as disclosed in WO 98/35958.

A preferred compound of formula I is PTK787. More preferably, PTK787 is employed in the form of its succinate salt.

Compounds that are also preferred for the treatment of MDS according to the present invention are those generically or specifically disclosed, mentioned or generically and specifically claimed in EP 1 259 487, WO 01/55114, EP 1 129 075, WO 00/27820, EP 1 107 964, WO 00/09495, EP 1 165 085, WO 00/59509, WO 02/090343, WO 01/85715, WO 01/85691, WO 02/092603, WO 03/040101 and WO 03/040102.

It will be understood that in the discussion of methods, references to the active, ingredients are meant to also include the pharmaceutically acceptable salts. If these active ingredients have, for example, at least one basic center, they can form acid addition salts. Corresponding acid addition salts can also be formed having, if desired, an additionally present basic center. The active ingredients having an acid group (for example COOH) can also form The term "treatment" as used herein comprises the treatment of patients having MDS or being in a pre-stage of said disease which effects the delay of progression of the disease in said patients.

For the treatment of MDS a 4-pyridylmethyl-phthalazine derivative can be administered alone or in combination with other forms of treatments, e.g. transfusions, bone marrow transplantation or administration of other therapeutic agents, e.g., vitamins such as vitamin D3 or vitamin A (retinoic acid), arsenic trioxide and/or those agents mentioned below.

To patients not producing enough red blood cells supportive treatment can be given in the form of transfusions. If patients are quite anemic (hematocrit consistently less than 25%), they will receive periodic transfusions, typically two units every two to six weeks. Alternatively, erythropoietin can be injected subcutaneously three to seven times a week.

If the bone marrow stain shows deposits of iron in the red cells indicating sideroblastic anemia, then the patient should take pyridoxine (Vitamin B6), e.g., 100 mg twice a day.

Too much iron that is deposited in the heart and liver is shortening life expectancy. In case of iron overload caused by red cell transfusions, a medicament lowering the iron load, e.g. DESFERAL^{™}, can be applied.

For patients with a low white cell count who have had at least one infection, it is worth trying growth-factor medication, i.e., granulocyte-colony stimulating factor (G-CSF, e.g. in the form as marketed under the trademark NEUPOGEN) and/or granulocyte macrophage-colony stimulating factor (GM-CSF, e.g., in the form as marketed under the trademark LEUCOMAX) is administered to such patients subcutaneously between one and seven times a week.

Hence, the present invention pertains also to a combination comprising a 4-pyridylmethyl-phthalazine derivative, preferably a compound of formula I as defined above, and at least one compound selected from the group consisting of vitamin A, vitamin B6, vitamin D3, arsenic trioxide, erythropoietin, a medicament lowering the iron load, e.g., DESFERALTM, G-CSF and GM-CSF, in which the active ingredients are present in each case in free form or in the form of a pharmaceutically acceptable salt and optionally at least one pharmaceutically acceptable carrier, for simultaneous, separate or sequential use, especially for use in a method of treating MDS. In one embodiment of the invention, in such combination a low dose of the 4-pyridylmethyl-phthalazine is applied together with at least one vitamin and/or growth factors such as erythropoietin or GM-CSF.

A combination comprising a 4-pyridylmethyl-phthalazine derivative and at least one compound selected from the group consisting of vitamin A, vitamin B6, vitamin D3, arsenic trioxide, erythropoietin, a medicament lowering the iron load, G-CSF and GM-CSF, in which the active ingredients are present in each case in free form or in the form of a pharmaceutically acceptable salt and optionally at least one pharmaceutically acceptable carrier, will be referred to hereinafter as a COMBINATION OF THE INVENTION.

The COMBINATION OF THE INVENTION can be a combined preparation or a pharmaceutical composition.

The term "a combined preparation", as used herein defines especially a "kit of parts" in the sense that the active ingredients as defined above can be dosed independently or by use of different fixed combinations with distinguished amounts of the ingredients, i.e., simultaneously or at different time points. The parts of the kit can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the active ingredients. The ratio of the total amounts of the active ingredient 1 to the active ingredient 2 to be administered in the combined preparation can be varied, e.g., in order to cope with the needs of a patient sub-population to be treated or the needs of the single patient which different needs can be due to age, sex, body weight, etc. of the patients. Preferably, there is at least one beneficial effect, e.g., a mutual enhancing of the effect of the first and second active ingredient, in particular a synergism, e.g. a more than additive effect, additional advantageous effects, less side effects, a combined therapeutical effect in a non-effective dosage of one or both of the first and second active ingredient, and especially a strong synergism the first and second active ingredient.

Additionally, the present invention provides a method of treating MDS comprising administering a COMBINATION OF THE INVENTION in an amount which is jointly therapeutically effective against MDS to a warm-blooded animal in need thereof.

The person skilled in the pertinent art is fully enabled to select relevant test models to prove the hereinbefore and hereinafter mentioned beneficial effects on MDS of a 4-pyridylmethyl-phthalazine derivative or of a COMBINATION OF THE INVENTION. The pharmacological activity of a 4-pyridylmethyl-phthalazine derivative or a COMBINATION OF THE INVENTION may, for example, be demonstrated in a suitable clinical study. Suitable clinical studies are, for example, open label non-randomized studies in patients with advanced MDS. Such studies prove in particular the synergism observed with the COMBINATIONS OF THE INVENTION. The beneficial effects on MDS can be determined directly through the results of such studies or by changes in the study design which are known as such to a person skilled in the art. For example, in a combination study one combination partner can be administered with a fixed dose and the dose of a second combination partner is escalated until the Maximum Tolerated Dosage (MTD) is reached. Alternatively, a placebo-controlled, double blind study can be conducted in order to prove the benefits of the COMBINATION OF THE INVENTION mentioned herein.

It is one objective of this invention to provide a pharmaceutical composition comprising a quantity, which is jointly therapeutically effective against MDS comprising the COMBINATION OF THE INVENTION. In this composition, the combination partners can be administered together, one after the other or separately in one combined unit dosage form or in two separate unit dosage forms. The unit dosage form may also be a fixed combination.

The pharmaceutical compositions for separate administration of the combination partners and for the administration in a fixed combination, i.e. a single galenical composition comprising at least two combination partners, according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application.

Novel pharmaceutical composition contain, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partners of the COMBINATION OF THE INVENTION may be administered simultaneously or sequentially and in any order, and the components may be administered separately or as a fixed combination. For example, the method of treatment of MDS according to the present invention may comprise (i) administration of a combination partner (a) in free or pharmaceutically acceptable salt form and (ii) adminstration of a combination partner (b) in free or pharmaceutically acceptable salt form, simultaneously or sequentially in any order, in jointly therapeutically effective amounts, preferably in synergistically effective amounts, e.g. in daily dosages corresponding to the amounts described herein. The individual combination partners of the COMBINATION OF THE INVENTION can be administered separately at different times during the course of therapy or concurrently in divided or single combination forms. Furthermore, the term administering also encompasses the use of a pro-drug of a combination partner that convert in vivo to the combination partner as such. The instant invention is therefore to be understood as embracing all such regimes of simultaneous or alternating treatment and the term "administering" is to be interpreted accordingly.

The effective dosage of a 4-pyridylmethyl-phthalazine derivative and of the combination partners employed in the COMBINATION OF THE INVENTION may vary depending on the particular compound or pharmaceutical composition employed, the mode of administration, the type of the MDS being treated, the severity of the MDS being treated and the co-medication. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of a 4-pyridylmethyl-phthalazine derivative or of the single active ingredients of the COMBINATION OF THE INVENTION required to prevent, counter or arrest the progress of the condition. Optimal precision in achieving concentration of the active ingredients within the range that yields efficacy without toxicity requires a regimen based on the kinetics of the active ingredients' availability to target sites.

If the warm-blooded animal is an adult human, the dosage of a compound of formula I, especially PTK787, is preferably in the range of about 200 to 2000, more preferably about 500 to 1800, and most preferably 800 to 1500, mg/day. Preferably, the total daily dosage of a compound of formula I, is applied to the warm-blooded animal by administration of two separate units comprising the same or different amounts of the compound of formula I, e.g. a total dosage of 1500 mg/day can be administered by giving the warm-blooded animal units of 750 mg twice daily.

When the combination partners employed in the COMBINATION OF THE INVENTION are applied in the form as marketed as single drugs, their dosage and mode of administration can take place in accordance with the information provided on the package insert of the respective marketed drug in order to result in the beneficial effect described herein, if not mentioned herein otherwise.

Moreover, the present invention provides a commercial package comprising as active ingredients the COMBINATION OF THE INVENTION, together with instructions for simultaneous, separate or sequential use thereof in the treatment of MDS.

The present invention also provides the use of a 4-pyridylmethyl-phthalazine derivative and the use of a COMBINATION OF THE INVENTION for the preparation of a medicament for the treatment of MDS.

### Example

Twelve patients having untreated advanced MDS received orally 500 or 750 mg PTK787 twice daily. Two patients with MDS showed stable disease with excellent performance status for a substantial period of time, namely one patient with baseline 18% bone marrow blasts has remained under 30% for 8 months and a second patient with baseline 11.5% blasts has remained ≤ 16% for 4 months.

The Example demonstrates that PTK787 slows disease progression in at least some patients with MDS.

## Claims

1. Use of a 4-pyridylmethyl-phtalazine derivative for the preparation of a medicament for the treatment of myelodysplastic syndromes.

2. Use according to claim 1 comprising administering a 4-pyridylmethyl-phtalazine derivative of formula I 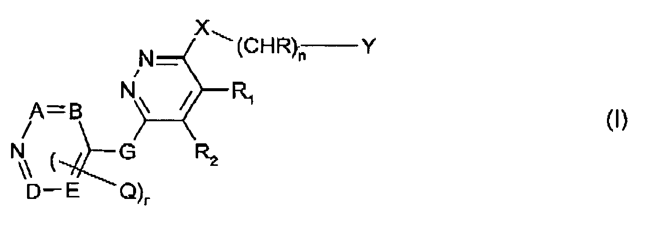 wherein
r is 0 to 2,
n is 0 to 2,
m is 0 to 4,
R₁ and R₂ (i) are
(i) together form a bridge in subformula I* 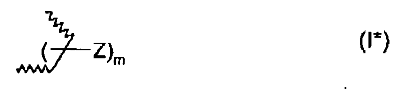 the binding being achieved via the two terminal carbon atoms, or
(ii) together form a bridge in subformula I** 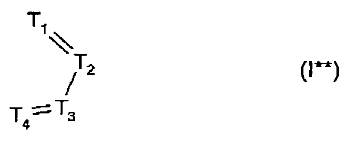
wherein one or two of the ring members T₁, T₂, T₃ and T₄ are nitrogen, and the others are in each case CH, and the binding is achieved via T₁ and T₄;
A, B, D, and E are, independently of one another, N or CH, with the stipulation that not more than 2 of these radicals are N;
G is lower alkylene, lower alkylene substituted by acyloxy or hydroxy, -CH₂-O- CH₂-S-, -CH₂-NH-, oxa (-O-), thia (-S-), or imino (-NH-);
Q is lower alkyl;
R Is H or lower alkyl;
X is imino, oxa, or thia;
Y is unsubstituted or substituted aryl, pyridyl, or unsubstituted or substituted cycloalkyl; and
Z is amino, mono- or disubstituted amino, halogen, alkyl, substituted alkyl, hydroxy, etherified or esterified hydroxy, nitro, cyano, carboxy, esterified carboxy, alkanoyl, carbamoyl, N-mono- or N,N-disubstituted carbamoyl, amidino, guanidino, mercapto, sulfo, phenylthio, phenyl-lower alkylthio, alkylphenylthio, phenylsulfonyl, phenyl-lower alkylsulfinyl or alkylphenylsulfinyl, substituents Z being the same or different from one another if more than 1 radical Z is present;
and wherein the bonds **characterized**, if present, by a wavy line are either single or double bonds;
or an N-oxide of the defined compound, wherein 1 or more N atoms cany an oxygen atom, or the salt of such compound having at least one salt-forming group,
to a warm-blooded animal in need thereof.

3. Use of claim 2 wherein the 4-pyridylmethyl-phthalazine derivative of formula I is 1-(4-chloroanilino)-4-(4-pyridylmethyl)phthalazine (PTK 787).

4. Use according to any one of claims 1 to 3 wherein the disease is resistant to conventional chemotherapy.

5. Use according to any one of claims 1 to 4 wherein the warm-blooded animal is a human.

6. Use according to any one of claims 1 to 5 wherein the total daily dosage of a compound of formula I is applied to the warm-blooded animal by administration of two separate units comprising the same or different amounts of the compound of formula I.

7. A combination comprising a 4-pyridylmethyl-phthalazine derivative and at least one compound selected from vitamin A. vitamin B6, vitamin D3, arsenic trioxide, erythropoietin, a medicament lowering the iron load, G-CSF and GM-CSF, in which the active ingredients are present in each case in free form or in the form of a pharmaceutically acceptable salt and optionally at least one pharmaceutically acceptable carrier, for simultaneous, separate or sequential use.

8. Combination according to claim 7 wherein the 4-pyridylmethyl-phthalazine derivative is 1-(4-chloroanilino)-4-(4-pyridylmethyl)phthalazine (PTK 787).

9. Combination according to claim 7 or 8 for simultaneous, separate or sequential use in the treatment of myelodysplastic syndromes.

10. Use of a combination as defined in claim 7 or 8 for the preparation of a medicament for the treatment of myelodysplastic syndromes.

11. A pharmaceutical composition comprising a quantity, which is jointly therapeutically effective against myelodysplastic syndromes, of a combination according to claim 7 or 8 and at least one pharmaceutically acceptable carrier.

12. A commercial package comprising a 4-pyridylmethyl-phthalazine derivative and at least one compound selected from vitamin A, vitamin B6, vitamin D3, arsenic trioxide, erythropoietin, a medicament lowering the iron load, G-CSF and GM-CSF, together with instructions for simultaneous, separate or sequential use thereof in the treatment of myelodysplastic syndromes.

## Patentansprüche

1. Verwendung eines 4-Pyridylmethyl-phthalazinderivats zur Herstellung eines Arzneimittels zur Behandlung von myelodysplastischen Syndromen.

2. Verwendung nach Anspruch 1, umfassend eine Verabreichung eines 4-Pyridylmethyl-phthalazinderivats der Formel I 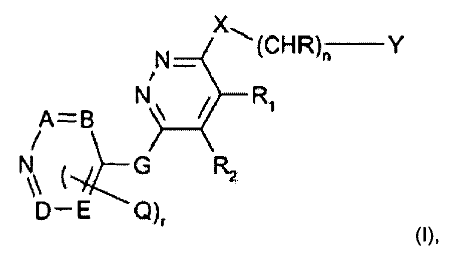 wobei
r für 0 bis 2 steht,
n für 0 bis 2 steht,
m für 0 bis 4 steht,
R₁ und R₂
(i) zusammen eine Brücke in der Unterformel I* bilden 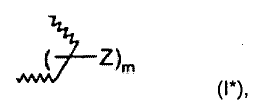 wobei die Bindung zustande gebracht wird über die zwei endständigen Kohlenstoffatome, oder
(ii) zusammen eine Brücke in der Unterformel I** bilden 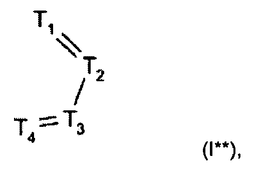
wobei eines oder zwei der Ringglieder T₁, T₂, T₃ und T₄ für Stickstoff steht und die anderen in jedem Fall für CH stehen, und die Bindung zustande gebracht wird über T₁ und T₄;
A, B, D und E unabhängig voneinander für N oder CH stehen, unter der Bedingung, dass nicht mehr als zwei von diesen Resten für N stehen;
G für Niederalkylen, durch Acyloxy oder Hydroxy substituiertes Niederalkylen, -CH₂-O-, -CH₂-S-, -CH₂-NH-, Oxa (-O-), Thia (-S-) oder Imino (-NH-) steht;
Q für Niederalkyl steht;
R für H oder Niederalkyl steht;
X für Imino, Oxa oder Thia steht;
Y für unsubstituiertes oder substituiertes Aryl, Pyridyl oder unsubstituiertes oder substituiertes Cycloalkyl steht;
und
Z steht für Amino, mono- oder disubstituiertes Amino, Halogen, Alkyl, substituiertes Alkyl, Hydroxy, verethertes oder verestertes Hydroxy, Nitro, Cyano, Carboxy, verestertes Carboxy, Alkanoyl, Carbamoyl, N-mono- oder N,N-disubstituiertes Carbamoyl, Amidino, Guanidino, Mercapto, Sulfo, Phenylthio, Phenylniederalkylthio, Alkylphenylthio, Phenylsulfonyl, Phenylniederalkylsulfinyl oder Alkylphenylsulfinyl, wobei die Substituenten Z gleich oder verschieden sind voneinander, wenn mehr als ein Rest Z vorliegt;
und wobei die Bindungen, die durch eine gewellte Linie **gekennzeichnet** sind, wenn vorliegend entweder Einfach- oder Doppelbindungen sind;
oder eines N-Oxids der definierten Verbindung, wobei 1 oder mehrere N-Atom(e) ein Sauerstoffatom trägt/tragen,
oder des Salzes einer solchen Verbindung, die mindestens eine salzbildende Gruppe aufweist,
an ein warmblütiges Lebewesen, das sie braucht.

3. Verwendung nach Anspruch 2, wobei das 4-Pyridylmethyl-phthalazinderivat der Formel I für 1-(4-Chloranilino)-4-(4-pyridylmethyl)phthalazin (PTK787) steht.

4. Verwendung nach einem der Ansprüche 1 bis 3, wobei die Erkrankung resistent ist gegenüber einer herkömmlichen Chemotherapie.

5. Verwendung nach einem der Ansprüche 1 bis 4, wobei das warmblütige Lebewesen für einen Menschen steht.

6. Verwendung nach einem der Ansprüche 1 bis 5, wobei die gesamte tägliche Dosierung einer Verbindung der Formel I an ein warmblütiges Lebewesen angewendet wird durch Verabreichung von zwei getrennten Einheiten, umfassend die gleichen oder verschiedenen Mengen der Verbindung der Formel I.

7. Kombination, umfassend ein 4-Pyridylmethyl-phthalazinderivat und mindestens eine Verbindung, die ausgewählt ist aus Vitamin A, Vitamin B6, Vitamin D3, Arsentrioxid, Erythropoietin, ein Arzneimittel, das die Eisenbeladung senkt, G-CSF und GM-CSF, wobei die wirksamen Inhaltsstoffe in jedem Fall in freier Form oder in der Form eines pharmazeutisch annehmbaren Salzes vorliegen, und gegebenenfalls mindestens einen pharmazeutisch annehmbaren Träger zur gleichzeitigen, getrennten oder sequenziellen Verwendung.

8. Kombination nach Anspruch 7, wobei das 4-Pyridylmethyl-phthalazinderivat für 1-(4-Chloranilino)-4-(4-pyridylmethyl)phthalazin (PTK787) steht.

9. Kombination nach Anspruch 7 oder 8 zur gleichzeitigen, getrennten oder sequenziellen Verwendung bei der Behandlung von myelodysplastischen Syndromen.

10. Verwendung einer Kombination wie in Anspruch 7 oder 8 definiert zur Herstellung eines Arzneimittels zur Behandlung von myelodysplastischen Syndromen.

11. Pharmazeutische Zusammensetzung, umfassend eine Menge, die gemeinsam therapeutisch wirksam ist gegen myelodysplastische Syndrome, von einer Kombination nach Anspruch 7 oder 8 und mindestens einen pharmazeutisch annehmbaren Träger.

12. Handelspackung, umfassend ein 4-Pyridylmethyl-phthalazinderivat und mindestens eine Verbindung, die ausgewählt ist aus Vitamin A, Vitamin B6, Vitamin D3, Arsentrioxid, Erythropoietin, einem Arzneimittel, das die Eisenbeladung senkt, G-CSF und GM-CSF, zusammen mit Anleitungen zur gleichzeitigen, getrennten oder sequenziellen Verwendung davon bei der Behandlung von myelodysplastischen Syndromen.

## Revendications

1. Utilisation d'un dérivé de 4-pyridylméthyl-phtalazine pour la préparation d'un médicament destiné au traitement des syndromes myélodysplasiques.

2. Utilisation selon la revendication 1, comprenant l'administration d'un dérivé de 4-pyridylméthyl-phtalazine de formule I 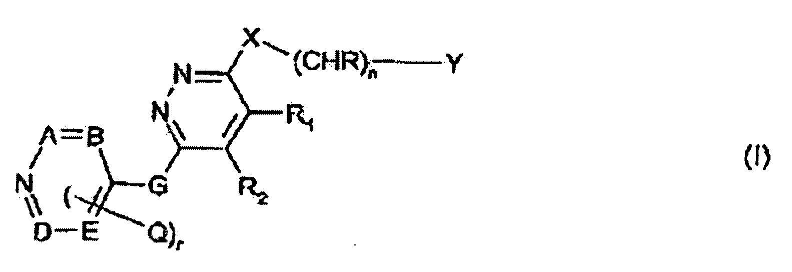 dans laquelle
r est égal à 0 à 2,
n est égal à 0 à 2,
m est égal à 0 à 4,
R₁ et R₂
(i) forment ensemble un pont dans la sous-formule I* 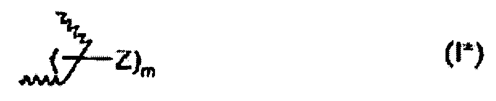 la liaison étant obtenue par le biais des deux atomes de carbone terminaux, ou
(ii) forment ensemble un pont dans la sous-formule I** 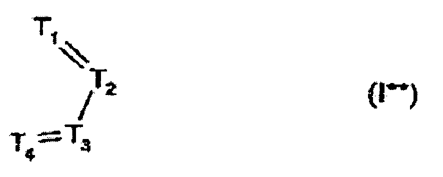
dans laquelle un ou deux des éléments de cycle T₁, T₂, T₃ et T₄ sont des atomes d'azote, et les autres représentent dans chaque cas CH, et la liaison est obtenue par le biais de T₁ et T₄;
A, B, D et E représentent, indépendamment les uns des autres, N ou CH, à condition que pas plus de 2 de ces radicaux ne représentent N;
G est un groupe alkylène inférieur, alkylène inférieur substitué par un groupe acyloxy ou hydroxy, -CH₂-O-, -CH₂-S-, -CH₂-NH-, oxa (-O-), thia (-S-) ou imino (-NH-);
Q est un groupe alkyle inférieur;
R représente H ou un groupe alkyle inférieur;
X est un groupe imino, oxa ou thia;
Y est un groupe aryle non substitué ou substitué, pyridyle, ou cycloalkyle non substitué ou substitué;
et
Z est un groupe amino, amino mono- ou disubstitué, un atome d'halogène, un groupe alkyle, alkyle substitué, hydroxy, hydroxy éthérifié ou estérifié, nitro, cyano, carboxy, carboxy estérifié, alcanoyle, carbamoyle, carbamoyle N-mono- ou N,N-disubstitué, amidino, guanidino, mercapto, sulfo, phénylthio, phénylalkyl(inférieur)thio, alkylphénylthio, phénylsulfonyle, phénylalkyl(inférieur)sulfinyle ou alkylphénylsulfinyle, les substituants Z étant identiques ou différents les uns des autres si plus d'un radical Z est présent;
et dans laquelle, si elles sont présentes, les liaisons **caractérisées par** une ligne ondulée sont des liaisons simples ou des doubles liaisons;
ou un N-oxyde du composé défini, où 1 ou plusieurs atomes de N portent un atome d'oxygène,
ou le sel d'un tel composé ayant au moins un groupe formant un sel,
à un animal homéotherme qui en a besoin.

3. Utilisation selon la revendication 2, dans laquelle le dérivé de 4-pyridylméthyl-phtalazine de formule I est la 1-(4-chloroanilino)-4-(4-pyridylméthyl)phtalazine (PTK787).

4. Utilisation selon l'une quelconque des revendications 1 à 3, où la maladie est résistante à une chimiothérapie classique.

5. Utilisation selon l'une quelconque des revendications 1 à 4, où l'animal homéotherme est un humain.

6. Utilisation selon l'une quelconque des revendications 1 à 5, où la posologie journalière totale d'un composé de formule I s'applique à l'animal homéotherme par administration de deux unités séparées comprenant des quantités identiques ou différentes du composé de formule I.

7. Combinaison comprenant un dérivé de 4-pyridylméthyl-phtalazine et au moins un composé choisi parmi la vitamine A, la vitamine B6, la vitamine D3, le trioxyde d'arsenic, l'érythropoïétine, un médicament réduisant la charge en fer, G-CSF et GM-CSF, dans laquelle les principes actifs sont présents dans chaque cas sous forme libre ou sous la forme d'un sel pharmaceutiquement acceptable et, éventuellement, au moins un véhicule pharmaceutiquement acceptable, pour une utilisation simultanée, séparée ou séquentielle.

8. Combinaison selon la revendication 7, dans laquelle le dérivé de 4-pyridylméthyl-phtalazine est la 1-(4-chloroanilino)-4-(4-pyridylméthyl)phtalazine (PTK787).

9. Combinaison selon la revendication 7 ou 8 pour une utilisation simultanée, séparée ou séquentielle dans le traitement des syndromes myélodysplasiques.

10. Utilisation d'une combinaison telle que définie dans la revendication 7 ou 8 pour la préparation d'un médicament destiné au traitement des syndromes myélodysplasiques.

11. Composition pharmaceutique comprenant une quantité, qui est conjointement thérapeutiquement efficace contre les syndromes myélodysplasiques, d'une combinaison selon la revendication 7 ou 8 et au moins un véhicule pharmaceutiquement acceptable.

12. Conditionnement commercial comprenant un dérivé de 4-pyridylméthyl-phtalazine et au moins un composé choisi parmi la vitamine A, la vitamine B6, la vitamine D3, le trioxyde d'arsenic, l'érythropoïétine, un médicament réduisant la charge en fer, G-CSF et GM-CSF, conjointement avec les instructions pour une utilisation simultanée, séparée ou séquentielle de ceux-ci, dans le traitement des syndromes myélodysplasiques.
